Europäisches Patentamt

European Patent Office

Office européen des brevets

(19)

(11) Publication number: **0 441 395 B1**

(12) # EUROPEAN PATENT SPECIFICATION

(45) Date of publication of patent specification: **03.05.95**

(51) Int. Cl.6: **C12P 21/08**, A61K 39/40, C12N 5/12, //C12N15/02, (A61K39/40,31:43),(A61K39/40, 31:70)

(21) Application number: **91101765.5**

(22) Date of filing: **08.02.91**

(83) Declaration under Rule 28(4) EPC (expert solution)

(54) **Human monoclonal antibody and pharmaceutical composition containing the same for the treatment of pseudomonas infections.**

(30) Priority: **08.02.90 JP 30020/90**

(43) Date of publication of application:
**14.08.91 Bulletin 91/33**

(45) Publication of the grant of the patent:
**03.05.95 Bulletin 95/18**

(84) Designated Contracting States:
**AT BE CH DE DK ES FR GB GR IT LI LU NL SE**

(56) References cited:
**EP-A- 0 256 713**
**EP-A- 0 341 684**
**EP-A- 0 383 090**

**JOURNAL OF CLINICAL MICROBIOLOGY, vol. 26, 1988; pp. 768-769&NUM;**

**JAPAN. JOURNAL OF EXPERIMENTAL MEDICINE, vol. 46, 1976; pp. 329-336&NUM;**

(73) Proprietor: **SUMITOMO PHARMACEUTICALS COMPANY, LIMITED**
**2-8, Doshomachi 2-chome,**
**Chuo-ku**
**Osaka 541 (JP)**

(72) Inventor: **Kohzuki, Tsuneo**
**2-3-21-108, Oogi,**
**Higashinada-ku**
**Kobe-shi,**
**Hyogo-ken (JP)**
Inventor: **Uezumi, Ikuko**
**5-1-30, Syuntoku-cho**
**Higashiosaka-shi,**
**Osaka-fu (JP)**
Inventor: **Irie, Kenji**
**2-25-8, Yuyamadai**
**Kawanishi-shi,**
**Hyogo-ken (JP)**
Inventor: **Ochi, Hiroshi**
**2-11-8-110, Sonehigashi-cho**
**Toyonaka-shi,Osaka-fu (JP)**

Rank Xerox (UK) Business Services
(3.10/3.09/3.3.3)

Inventor: **Horigome, Kazuhiko**
**2-14-7, Mefu**
**Takarazuka-shi,**
**Hyogo-ken (JP)**
Inventor: **Noguchi, Hiroshi**
**4-4-153, Seiwadainishi**
**Kawanishi-shi,**
**Hyogo-ken (JP)**

(74) Representative: **VOSSIUS & PARTNER**
**Postfach 86 07 67**
**D-81634 München (DE)**

**Description**

This invention relates to a human monoclonal antibody to Pseudomonas aeruginosa (hereinafter, referred to as "P. aeruginosa"), obtainable from the hybridoma cell line having the deposit accession number FERM BP-3213 and the production and the use thereof. More particularly, it relates to a human monoclonal antibody having the following characteristics;

1) the antigenic determinant for the monoclonal antibody is an O-antigen of P. aeruginosa;

2) the monoclonal antibody is prophylactically and therapeutically effective on infections caused by P. aeruginosa;

3) the monoclonal antibody shows synergistic effects in the treatment of experimental P. aeruginosa infections when used in association with carbapenem or aminoglycoside antibiotics; and

4) the monoclonal antibody belongs to the IgM (Immunoglobulin M) class.

This invention also relates to the use of the monoclonal antibody, and the hybridoma cell line capable of producing the monoclonal antibody and the process for the production of the antibody by culturing the hybridoma cell line.

The human monoclonal antibody of the invention has proved to be effective for the prevention and the therapy of bacterial infections and/or endotoxin shocks caused by P. aeruginosa.

The kinds of bacteria causing infectious diseases, i.e. infection-causing bacteria, have changed with development of antibiotics as clinically used. As the result, infectious diseases caused by bacteria which used to show only low pathogenicity or virulence have increased. Thus, P. aeruginosa is currently one of the major pathogenic bacteria causing infectious diseases, of which serious symptoms often lead patients to death, particularly when their immunological competence is low due to continuous administration of immunosupressants or when they suffer from immunodeficiency or immunodepression diseases caused by cancer or burn or the like.

Among currently used prophylactic or therapeutic methods, the most popular one is chemotherapy wherein antibiotics and/or synthetic antimicrobial agents are used. A number of antibiotics which have a marked inhibitory effect against almost all of the gram-positive bacteria such as Staphylococci and most of gram-negative bacteria such as Escherichia coli have been developed so far. Although these antibiotics possess potent antimicrobial activity, they are not necessarily effective on serious infections as is often seen in animal models which have been infected with a large amount of bacteria. It is often said that the therapeutic effects of a treatment which uses only one antibiotic is limited to some extent, even in high-dose therapy.

As to the infections caused by strains of P. aeruginosa, there are only a few drugs to which said organisms are susceptible. Furthermore, strains of P. aeruginosa have a tendency to gain tolerance to newly developed antibiotics against P. aeruginosa.

The other therapeutic method is antibody therapy wherein antibodies such as immunoglobulin preparations are used in association with antibiotics. A serum of high antibody titer can be obtained by active immunization of animals such as horse or rabbit and antibody therapy can be made by administration of such serum. The remarkable therapeutic effects of such a serum have been proved on experimental infections using various animals.

It is known from the cases of diphtheria toxin and viper toxin that antibody therapy by sera originating from animals are also effective on human beings. However, this therapy cannot be applied commonly to all bacterial infections because of the danger of causing serious side-effects such as allergic reactions including anaphylaxis.

Conventional human immunoglobulin preparations have been manufactured by collecting blood from healthy persons or those who had been infected with P. aeruginosa in the past, subjecting the blood to fractionation to obtain an immunoglobulin fraction, purifying the fraction and eliminating agglutinating materials therefrom by addition of polyethylene glycol, treatment with protease, sulfonization, DEAE column chromatography, etc., which is followed by formulation of the resulting product into intramuscularly or intravenously injectionable preparations. Although these preparations are advantageous in not causing anaphylaxis or any other side-effects, they still have several drawbacks.

First, due to their relatively low anti-bacterial antibody titer, sufficient therapeutic effects may not necessarily be produced. Second, the stable supply of immunoglobulin preparations with a high antibody titer in a large amount is not easy. Since blood is collected from unknown healthy or diseased persons, it is extremely difficult to obtain serum of high titer constantly, which results in final products with variously changed antibody titer from lot to lot. Third, they may be contaminated with hepatitis virus such as HB virus or adult T cell leukaemia virus (ATLV) because they are made from randomly collected human sera. In order to overcome these drawbacks, production of human monoclonal antibodies which are reactive with P.

3

aeruginosa and useful for the prophylaxis and treatment of infections with P. aeruginosa is highly desired.

When an antibody is bound to the surface of a bacterial cell, the phagocytosis of macrophages on the bacterial cell is accelerated (i.e., acceleration of phagocytosis due to opsonization), or the lysis of the bacterial cell by complement takes place. Examples of surface antigens of P. aeruginosa which can serve as targets of antibody therapy include lipopolysaccharide (LPS), outer membrane protein, flagellum, pilus, and the like.

LPS consists of O-polysaccharide which represents the O-antigen, an outer core oligosaccharide which is common among species to some extent, an inner core oligosaccharide which is almost generally common to all enterobacteria, and lipid A. The O-polysaccharide antigen (O-antigen), which exists at the utmost outer surface of a bacterial cell, consists of repeating units consisting of polimerized 2 - 5 sugar residues and its structure varies to a large extent. The structures of almost all O-antigens of standard serotype strains of P. aeruginosa have already been determined (Eur. J. Biochem., 106, 643-651, 1980; Eur. J. Biochem., 125, 229-237, 1982; Eur. J. Biochem., 150, 541-550, 1985; Eur. J. Biochem., 155, 659-669, 1986; Eur. J. Biochem., 167, 549-561, 1987).

Because the determination of the structures of O-antigens requires a lot of time and labor, antisera or mouse monoclonal antibodies against O-antigens from the standard strains are employed for the classification of the strains of P. aeruginosa. For example, an unknown strain of P. aeruginosa is classified in accordance with its immunoreactivity with a known antibody or antiserum. This classification is known as a serotype, and typical examples of the serotype classification are as follows: Types 1 to 17 according to the classification by Homma et al. (Japan. J. Exp. Med., 44, 1 (1974); Types 1 to 17, according to the classification by Fisher et al. (J. Bacteriol., 98, 835 (1969)); Types A to M according to the classification by the Serotyping committee for the Japan Pseudomonas aeruginosa Society (hereinafter, referred to as "Japanese Committee") (Japan. J. Exp. Med., 46, 329 (1976)); Types 1 to 17 according to the classification by International Antigenic Typing System (IATS) (Int. J. Syst. Bacteriol., 33, 256-264 (1983)), etc..

It is well known that an antibody specific for a certain O-antigen shows a strong prophylactic or therapeutic effect on infections caused by P. aeruginosa strains of a serotype to which said O-antigen belongs but does not show any effect against infections caused by strains of any other serotype.

It is known to those skilled in the art that a cell line capable of producing a specific antibody can be prepared by a transformation method employing EB virus or the cell fusion method. These techniques are also applicable to a production of a human monoclonal antibody specific for the O-antigen of lipopolysaccharides of P. aeruginosa. For example, Japanese Patent Publication (KOKAI) No. 69796/1986 describes a production of transformants which produce human monoclonal antibody specific for an O-antigen of P. aeruginosa by transforming spleen lymphocytes derived from a person who had been infected with P. aeruginosa in the past with EB virus. Japanese Patent Publication (KOKAI) No. 152281/1986 discloses the preparation of human-mouse hybridoma cell lines producing a human monoclonal antibody specific for an O-antigen of P. aeruginosa by means of cell fusion in which a mouse myeloma cell (P3-X63-Ag8-U1 strain) was fused with a pokeweed mitogen-primed tonsillar cell derived from a patient of tonsillitis.

A general description about the combined treatments of antibodies and antibiotics can be seen in the above mentioned publications. Such treatments also can be seen in the Japanese Patent Publication (KOKAI) 107999/1988 with specific examples of antibiotics. However, no report has provided therapeutically useful combinations of particular antibodies and antibiotics. As the clinical application of the combined treatment of antibiotics and globulin preparations such as monoclonal antibody is believed to increase, it is important to know which antibiotics should be used in combination with a given antibody to obtain high therapeutic effects.

Combined treatment with antibiotics and specific antibodies have been shown specifically using polyclonal globulin preparations (IgG) as follows. Traub et al., Chemotherapy (Basel), 35, 23-38, 1989, reported that the effect appears to be dependent more on the strains of the pathogen than on the antibiotics. Thus, a certain strains is susceptible to almost all treatments, but others are not. Sunagawa et al. (Annual reports of the J. Japan. Asoc. Infect. Dis., 56, 1196-1202, 1982) reported that the therapeutic effects obtained using cephalosporin, penicillin, or an aminoglycoside antibiotic in association with a certain antibody were almost the same. These reports however failed to show any particular antibiotics capable of enhancing the effects of combined treatment. This can be attributed to the fact that the antibodies used were polyclonal antibodies, especially those of the IgG class. It can be also noted that Traub, who administered a high dose of antibody, i.e., 500 mg/kg, achieved greater therapeutic effects than Sunagawa, who employed a lower dose, i.e., 100 mg/kg. These results demonstrate that it is difficult to obtain desired effects by the use of polyclonal antibodies at their clinically acceptable dose (50 - 100 mg/kg).

There have been reported combined treatments which use monoclonal antibodies in association with antibiotics. The Japanese Patent Publication (KOKAI) No. 10022/1987 concluded that the mouse IgG monoclonal antibody specific for O-antigen of P. aeruginosa shows a synergistic effect when used together with astromycin (aminoglycoside antibiotic). It also describes that other antibiotics such as β-lactam antibiotics including cephalosporins are usable instead of astromycin, although there are no practical examples. Tsuji (in the Annual Reports of the 23rd. Meeting of the Japan Pseudomonas aeruginosa Society, 8 - 15, 1989) reported that a combined treatment of antibiotics and a monoclonal antibody specific for O-antigen of P. aeruginosa is additive. Young et al. (Rev. Infect. Dis., 11, suppl. 7, s1564 - s1571) reported that the combined use of antibiotics and a monoclonal antibody specific for the core antigen of E. coli is not significantly effective.

These previous studies have never been employed monoclonal antibodies of the IgM class. The results of these studies vary so much from each other that one can hardly select an appropriate antibiotic to be used in association with an IgM monoclonal antibody.

Bacterial infection is often followed by what is called "endotoxin shock". Typical symptoms of the endotoxin shock are fever, hypotension, shock, and death.

It was reported that various antisera and monoclonal antibodies specific for core glycolipid of Gram-negative bacteria are effective in prevention of an endotoxin shock (WO 8404458; WO 8501659; EP-A-0174204; Japanese Patent Publication (KOKAI) No. 130300/1986; Infect. Immun., 45, 631-636, 1984; Proc. Natl. Acad. Sci., USA, 82, 1790-1794, 1985; J. Infect. Dis., 151, 1005-1011, 1985; J. Infect. Dis., 136 - (Suppl.), S167-173, 1977; Infect. Immun., 46, 677-681, 1984; Clin. Invest., 72, 1874-1881, 1983; Clin. Res., 30, 522A, 1982; Clin. Res., 32, 518A, 1984; J. Infect. Dis., 159, 641-647, 1989; J. Urol., 141, 1463-1466, 1989; J. Exp. Med., 171, 889-896, 1990).

The above-mentioned antisera or monoclonal antibodies, which are prepared on the basis of the core glycolipid antigen, bind to various Gram-negative bacteria. This apparently shows that the associated epitope exists in an inner core moiety comprising heptose, KDO, etc., or in the lipid A moiety. It is known that the lipid A moiety is essential for the endotoxin activity and is a toxophore itself. The anti-core antibody seems to bind specifically to this lipid A part of the molecule and blocks the biological activity of endotoxin thereby. However, there is no report that anti-O antigen antibody, whose anti-infectious effects depend mainly on its opsonization activity, is also effective against an endotoxin shock.

Accordingly, the technical problem underlying the present invention was to provide human monoclonal antibodies effective for preventing and treating infections and/or endotoxin shock caused by P. aeruginosa and to provide immunoglobulin preparations containing said monoclonal antibody and a method for producing the antibody constantly on a large scale. The solution of this technical problem was achieved by establishing cell lines producing a human monoclonal antibody specific for the O-antigen of P. aeruginosa by the use of human B lymphocytes, which antibody is obtainable from the hybridoma cell line having the deposit accession number FERM BP-3213, belongs to the IgM class and shows prophylactic and therapeutic effects on infections caused by P. aeruginosa. The human monoclonal antibody of the present invention is prophylactically and therapeutically effective against endotoxin shock. As the second step, the present inventors demonstrated unexpectedly high therapeutic effects of combined treatments of given antibiotics and the human monoclonal antibody (IgM class) of the present invention. It has been revealed that this monoclonal antibody exhibits unexpectedly strong therapeutic effects when used together with certain antibiotics.

More specifically, the human monoclonal antibody of the present invention is reactive with strains of P. aeruginosa of serotype E as classified by the Japanese Commitee's Classification, said antibody being therapeutically effective on experimental P. aeruginosa infections in mouse, and being synergistically effective when used together with aminoglycoside or carbapenem antibiotics on the same experimental infections. The human monoclonal antibody of the invention is distinguishable from those previously obtained ones in the following features;

(1) it belongs to the human IgM class; and

(2) it shows a synergistic effect when used in association with aminoglycoside or carbapenem antibiotics, while it shows an additive effect when used together with cepharosporin, penicillin or monobactam antibiotics. Therefore, according to the present invention, there can be obtained novel, significantly useful prophylactic and therapeutic methods for the treatment of P. aeruginosa infections.

Thus, the present invention provides a human antibody recognizing the O-antigen of P. aeruginosa and expressing a synergistic effect when used together with certain antibiotics. More specifically, it provides a monoclonal antibody specific for a unique antigenic determinant of P. aeruginosa, and its use and production. The present invention also provides a cell line capable of producing said specific antibody continuously, the methods for preparing said cell line and the process for producing the specific antibody by culturing the cell line.

The human cell line capable of producing continuously a human monoclonal antibody reactive with the O-antigen of LPS of P. aeruginosa can be established using any of known processes, as follows.

The first method comprises a transformation of human B lymphocytes sensitized in vivo or in vitro with P. aeruginosa (living bacteria or bacteria killed with formalin or by heating) or LPS derived from P. aeruginosa, with Epstein-Barr virus (hereinafter, referred to as EB virus) by mixing them together. The resultant transformed cells can grow continuously. Before or after the cloning, transformed cells are screened for the production of antibodies having the above-mentioned specific reactivity, and cultured in vitro to establish cell lines capable of producing the desired antibody continuously. The screening was performed by, for example, enzyme linked immunosorbent assay (ELISA) using a bacterial screening panel containing strains of 17 serotypes of P. aeruginosa.

The second method comprises a cell fusion of the sensitized human B lymphocyte as described above with a myeloma or B lymphoblastoid cell to establish a cell line which grows continuously in vitro and produces LPS-specific antibody as mentioned above.

The third method comprises a cell fusion of myeloma or B lymphoblastoid cells with EB-virus transformed cells as prepared in the first method to establish a cell line which grows continuously in vitro and produces LPS-specific antibody. The resulting cell lines produce antibodies when they are grown in vitro or in vivo (e.g., in the peritoneal cavity of a nude mouse). The monoclonal antibody of the invention can be obtained in large amounts by purifying the antibody secreted into the culture medium or ascites.

The human monoclonal antibody of the invention can be prepared according to the following steps.

(1) The preparation of antigen-sensitized human B lymphocytes.

(2) The establishment of cell lines capable of producing a monoclonal antibody by immortalizing the cells as prepared in (1).

(3) The cultivation of the cell lines as established in (2).

(4) The purification of specific monoclonal antibody from the culture as obtained in (3).

(5) The production of an immunoglobulin preparation of high titer, which preparation contains monoclonal specific antibody as purified in (4).

Each of these steps will be hereinafter explained in detail.

Step (1): -

The human B lymphocytes which can be used in the process are human lymphocytes which produce an antibody reactive with LPS of P. aeruginosa. Such cells can be separated from peripheral blood by centrifugation in the presence of a lymphocyte separation liquid such as Lymphoprep$^R$ or Mono-Poly Resolving Medium$^R$ (Flow Lab.). There may be also used B lymphocytes originating from tissues or organs (for example, lymphnode, spleen, and the like) extracted for the purpose of diagnosis or therapy of diseases, umbilical cord blood, and the like. It is preferable to obtain an in vivo immunized-lymphocyte, which can be obtained for example, from a person who was infected with P. aeruginosa in the past. Pertinent persons, the donors of lymphocytes, can be chosen by previous measurement of the antibody titer in their sera. Alternatively, human B lymphocytes may be obtained from any person irrespective of their medical history in the past. The resulting lymphocytes are then subjected to an in vitro immunization by mixing with an antigen such as inactivated P. aeruginosa or LPS thereof. Further, solutions containing lymphokines such as B cell proliferation factors (e.g., plant lectins such as pokeweed mitogen (PWM), bacterial components such as Cowan I, human lymphocyte mixed culture, spleen, thymus or umbilical cord blood cell culture) may be added to human B lymphocytes for sensitization in vitro, followed by proliferation and differentiation to give antibody-producing cells. The thus obtained human B lymphocytes carrying antibody molecules at the cell surface can secret only a small amount of antibody for a certain limited period but they are not immortal.

Step (2): -

For changing the above sensitized human B lymphocytes to continuously and unlimitedly proliferable cell lines (immortal cell lines), essentially two methods can be employed in the present invention.

The first method comprises infecting the sensitized B lymphocyte with EBV, which has been prepared from the marmoset cell B95-8, by shake culture. Preferably, 2 - 10 $TD_{50}$ of virus (the $TD_{50}$ value will be defined hereinafter) is mixed with one cell. Thus, the lymphocytes are plated on a 96 well microplate at a density of 0.5 - 3 x $10^4$ cells/well. By the use of a preselected conventional medium such as RPMI1640 medium or Eagle's MEM containing 2 - 20 % (v/v) fetal bovine serum, the lymphocytes are cultured at 32 - 37 °C for 2 - 5 weeks in the presence of 5 - 10 % $CO_2$ to obtain transformed cells. During the culture, half of the medium is changed every 2 or 4 days. If necessary, 1 - 2 $\mu$g/ml of cyclosporin A, antibiotics, or synthetic antimicrobes which prevent the infection by mycoplasma is added to the medium. Ten days after infection, cell populations of 20 - 200 transformed cells are optical-microscopically observed and can be distinguished from non-transformed cells. The culture supernatants from the wells which contain well-grown transformed cells are tested for the antibody titer by ELISA to select wells expressing high antibody titer. The desired transformants from the selected wells are then subjected to cloning. The cloning is conducted by the use of the limiting dilution method or the soft agar method. In case of the limiting dilution method, the cluster of transformed cells is scattered by repeated inhaling and exhaling of a solution containing the cluster with a pipette, diluted with the culture medium to an appropriate concentration, and planted and cultured on a 96 well microplate at a density of 0.5 - 100 cells/well. In case of the soft agar method, about 7 x $10^4$ - 7 x $10^5$ transformed cells are plated on a plate (30 mm∅) containing 0.36 - 0.4 % (w/v) of soft agar (preferably, sea plaque agarose) and the plate is incubated at 37 °C, in the presence of 5 % $CO_2$ to obtain colonies, each of which originates from a single cell. In the cloning, it is preferred to use mouse peritoneal cells, human umbilical cord blood lymphocytes, or X-ray treated mouse spleen cells as feeder layers. The antibody titer of culture supernatants of cloned cell lines is measured by ELISA which uses as a solid antigen, seventeen different serotypes of P. aeruginosa, and clones producing abundant specific antibody are selected. The cloning process and the selection of high titer antibody-producing clones are repeated two or three times, and there is established a transformed cell line which shows rapid growth, and abundant and steady production of specific antibody.

The second method comprises a cell fusion of the antigen-sensitized human B lymphocyte in the presence of polyethylene glycol (PEG). Examples of myeloma cells employable in the cell fusion include hypoxanthine-guanine phosphoribosyl transferase (HGPRT)-deficient mutants originating from mouse myeloma cells such as P3X63-Ag 8 (P3) or P3X63-Ag 8.653, HGPRT-deficient mutants originating from the human myeloma cell U-266, HGPRT-deficient mutants originating from mouse-human heteromyeloma cells such as SHM D-33. HGPRT-deficient mutants originating from human B lymphoblastoid cell can be used in place of myeloma cells.

As for the polyethylene glycol (PEG), there may be used, for instance, PEG 1,000 to 6,000 in a concentration of 30 to 50 % (w/v). The fusion efficiency can be enhanced by incorporation of lectin, poly-L-lysine, dimethylsulfoxide, etc. thereto.

The fusion may be carried out, for instance, in the same manner as described in the Köhler et al. (Nature, 256, 495 (1975)) wherein mouse cells are fused to obtain a hybridoma producing a mouse monoclonal antibody. For instance, the antigen-sensitized human lymphocytes and HGPRT-deficient myeloma cells or human-mouse heteromyeloma cells are mixed together in a proportion of 10 - 1:1, and 30 - 50 % (w/v). PEG 4000 is added portionwise thereto over a period of 0.5 to 1 minute, and the resultant mixture is allowed to stand for 1 to 10 minutes. To the mixture, 9 to 50 ml of a serum-free culture medium is added over a period of 5 to 10 minutes, and subsequently culture medium is added to obtain a cell suspension of $10^5$ - $10^6$ cells/ml. The cell suspension thus obtained is inoculated into a 96 well microtiterplate at a density of 2 x $10^4$ to 2 x $10^5$ cells per well. On the next day, half the amount of the medium is replaced by a hypoxanthine-aminopterin-thymidine-containing medium (HAT medium) or a hypoxanthine-azaserine-containing medium (HAz medium), and cultivation is effected at 32 to 37 °C in 5 % $CO_2$. For about 10 to 20 days, the culture medium is replaced by HAT medium, and subsequently by hypoxanthine-thymidine-containing medium (HT medium) for about 3 to 5 days. The replacement was done on half amount basis at intervals of 3 days for 2 or 3 weeks to obtain a proliferating colony, i.e. a hybridoma. It is also possible to select a hybridoma by the combined use of metabolism inhibitors without employing a HGPRT-deficient mutant.

The antibody titer of the culture medium containing a hybridoma is measured by the above-mentioned ELISA and the desired cell producing specific antibody is selected. The specific reactivity of the selected cell with LPS is determined by the Western blotting method. Cloning is repeated two or three times by the limiting dilution method or the soft agarose method to obtain a stable cell line having a high-rate proliferative property and a productivity of an antibody with high specificity. In these procedures, EB virus-transformed cells prepared in the 1st step can be used instead of sensitized human B lymphocytes.

The thus established cell lines obtained from antigen-sensitized human B lymphocytes according to the above-mentioned "EBV transformation method" and "EBV-hybridoma method" are characterized in that they are capable of continuously growing and steadily producing abundant amounts of specific antibody.

Step (3): -

The established hybridomas (0.5 - 5 x $10^5$ cells/ml) are cultured in a settled culture or spinner culture using a usual culture medium for animal cells in a vessel such as a cell culture flask or plate by the use of a $CO_2$ incubator at 32 to 37 °C under 2 to 10 % $CO_2$. Particularly when a culture is made at a large scale, a jar fermenter, a hollow fiber system or the like designed for animal cells may be used. The culture medium which can be used in the present procedure is an ordinary one (for instance, RPMI1640 or Eagle's MEM) containing 2 to 20 % serum of bovine fetus, calf, cow, horse, human or the like, a serum-free medium containing supplements required for the growth of cells (e.g. insulin, transferrin, ethanolamine, selenite, bovine albumin, lipid) or the like.

Step (4): -

Purification of the antibody may be carried out by conventional biochemical procedures (e.g. ammonium sulfate precipitation, ethanol precipitation, PEG fractionation, ion exchange chromatography, gel filtration, affinity chromatography, high performance liquid chromatography, electrophoresis). In the purification process, care should be taken for preventing the production of agglutination or the depression of antibody activity. For this purpose, human serum albumin (HSA) may be added in an amount of 0.05 to 2 %. Addition of amino acids such as glycine or alanine, especially basic amino acids such as lysine, arginine or histidine, carbohydrates such as glucose or mannitol, salts such as sodium chloride, etc. may be sometimes preferred. Agglutination, which tends to occur particularly in case of IgM antibody, can be prevented by the treatment with $\beta$-propionolactone, acetic anhydride or the like. Through the anti-agglutination treatment, an intravenously injectionable preparation can be obtained.

Step (5): -

The purified monoclonal antibody may be formulated into a biological preparation by a per se conventional procedure comprising, for instance, filtering though a membrane filter for removal of bacteria, admitting into sterilized vials with stabilisators and lyophilyzing.

The human monoclonal antibody of the present invention binds to the surface of a P. aeruginosa cell, through the reaction with the O-antigen, which leads to the opsonization of the P. aeruginosa cell. The opsonization enhances phagocytosis and bacteriolytic actions of phagocytes on the cell and activation of complement which accelerates the lysis of the cell. Accordingly, experimental mouse infections with P. aeruginosa can be treated by administration of the human monoclonal antibody of the invention.

It has been found that the human monoclonal antibody of the present invention showed synergistic effects when used together with carbapenem or aminoglycoside antibiotics in the treatment of experimentally infected mice , while it showed only additional effects when used together with cepharosporin, monobactam or penicillin antibiotics. This suggests that the present invention provides the solution to the problem of the "limitation" in the treatment of bacterial infections, a problem which was never solved by conventional methods where only either antibiotic or globulin preparation was used. Specifically, the solution has been established by choosing appropriate combinations of antibiotics and globulin preparations so as to obtain unexpectedly high therapeutic effects.

It has been believed that the therapeutic effects of the combined treatments with antibiotics and globulin preparations should be lowered when the administration of the latter is delayed (Watanabe, J. Japan. Asoc. Infect. Dis., 61, 1091-1101, 1987). Nevertheless, the human monoclonal antibody of the present invention, when used together with the Imipenem Cilastatin mixture, showed an improved therapeutic effect on experimental infection in the mouse even if the administration of the antibody was postponed for a certain period . This means that the therapeutic effects of the combined treatment of the invention will not be affected by the delay of administration of antibody by some reasons, for example, by diagnosis.

In the clinical use, the combined treatment may comprise one (or two) administration(s) of an antibiotic and one combined administration of an antibiotic and antibody, because the ordinary daily administration time for antibiotics is twice (or thrice) and that for globulin preparations is once. It can also be conducted by administering the antibody between two antibiotic-therapies subject to that the time lag is settled appropriately.

The human monoclonal antibody of the invention has opsonization activity as previously described. However, the mechanism by which the combined treatment of said antibody and imipenem preparation effects is not attributable to the enhancement of opsonization activity by the monoclonal antibody.

In addition, the human monoclonal antibody of the present invention which is reactive with the O-antigen showed a prophylactic and therapeutic activity in the treatment of an experimental endotoxin shock in the mouse. Therefore, the antibody is also useful in the prophylaxis and treatment of Pseudomonas-induced endotoxin shock.

For prevention and treatment of a P. aeruginosa infections or mixed infections by bacteria containing P. aeruginosa, the human monoclonal antibody of the invention may be administered to an adult patient in an amount of about 0.5 to 500 mg, preferably 5 to 50 mg. Preferred examples of antibiotics which can be used together with the human monoclonal antibodies include carbapenem antibiotics such as imipenem cilastatin mixture and/or aminoglycoside antibiotics such as gentamycin, tobramycin, sisomycin, and the like. Further advantages of the present monoclonal antibodies are shown below.

Since it is a protein of human-origin, side effects (e.g. anaphylaxis) as seen on the administration of a heterogenic protein do not occur. Furthermore, it being produced from a certain specific cell line, the possibility of contamination with unknown biohazardous materials is much smaller in comparison with conventional immunoglobulins prepared from a human blood originating from a number of unknown persons. The human monoclonal antibody of the invention is stably produced in vitro with a high antibody titer and in a large amount with easy quality control through a more advantageous production process than conventional ones which use human blood as the starting material.

The present invention will be hereinafter explained in detail by way of examples, but is not limited to those examples.

Example 1

Establishment of a human monoclonal antibody-producing clone by the EBV transformation method

(1) Preparation of the EBV solution and measurement of viral titer

Marmoset cells B95-8 which produce and release EBV were suspended in RPMI 1640 medium containing 10 % (v/v) fetal calf serum (FCS) at a density of $6.5 \times 10^5$ cells/ml. The suspension in a culture flask T-75 (Corning #25110) was stationarily cultured in the presence of 5 % $CO_2$ at 37 °C for 4 days. The culture supernatant was centrifuged for 10 minutes using a low-speed centrifuge (Tomy-Seiko RS-20BH) at 2,000 rpm (Rotor TS-7). The resultant supernatant was filtered through $0.45\mu m$ membrane filter (Milex SLHA0250S). The filtrate was charged in a Serum tube (Sumitomo Bakelite MS-4505), stored at -80 °C, and used for EBV transformation of human B lymphocytes. The virus titer of the EBV solution is determined substantially in accordance with the method of D. J. Moss and J. H. Pope (J. General Virology, 17: 233, 1972) using human peripheral blood lymphocytes as indicator cells. The suspension of human peripheral blood lymphocytes ($10^6$ cells/ml) in a RPMI 1640 medium containing 15 % FCS was dispensed in 96 well microtiterplates (Falcon #3040) at a rate of 100 $\mu l$/well. To the wells were added 20 $\mu l$ each of EBV solutions diluted sequentially by ten folds with the same medium. The rate of dilution ranged from $10^0$ to $10^7$. Each diluted solution was applied to six wells and the plate was incubated at 37 °C under $CO_2$. The incubation was continued for three weeks with replacing one third of the culture medium every 3 days. The resulting culture was screened for the presence of transformed cells by an optical microscope and the transformation rate was obtained for each dilution rate, from which was determined the maximum rate effective to transform 50 % of cells according to the Reed and Muench method. The $TD_{50}$ was then calculated inversely form the maximum rate and the amount of virus in the original sample was expressed by the $TD_{50}$ value. EBV solutions containing $10^5$ -$10^7$ $TD_{50}$ of virus per ml were obtained.

(2) Measurement of anti-P. aeruginosa antibody titer by ELISA

The antibody titer against P. aeruginosa surface antigen was measured as follows. P. aeruginosa was suspended in a phosphate buffered saline (pH 7.2; comprising NaCl (8 g/l), KCl (0.2 g/l), $NaHPO_4.12H_2O$ (2.99 g/l) and $KH_2PO_4$ (0.2 g/l); hereinafter, referred to as PBS) to give absorbance of 0.2 at a wavelength of 600 nm. The suspension was filled into 96 well microtiterplates (Falcon #3912) at 50 $\mu l$/well, followed by centrifugation at 2,000 rpm for 15 minutes. 2% Glutaraldehyde was added to each well at a rate of 50 $\mu l$/well to fix the bacterial cells to the microtiterplate. After removal of the bacterial suspension from the microtiterplates, a PBS solution containing 3% bovine serum albumin (BSA) was charged to the microtiterplate at a rate of 120 $\mu l$/well and incubated at 37 °C for 30 minutes for the purpose of blocking the unbound portions on the assay plate. The resulting microtiterplate was used as the antigen-coated plate in the subsequent operation. When desired, storage of such microtiterplate may be made at -20 °C.

Prior to the assay, the microtiterplate was washed with a 0.05 % Tween 20-containing PBS solution (PBST) three times. PBST was charged into wells at a rate of 50 $\mu$l/well, and a sample (serum, ascites or culture supernatant), optionally diluted with PBS, was added thereto at a rate of 50 $\mu$l/well, followed by incubation at 37 °C for 2 hours. The sample was removed from the plate, which was washed with PBST three times. Alkaline phosphatase-conjugated affinity purified goat anti-human immunoglobulin antibody (Kirkegaard & Perry Lb. Inc.) (2ndary antibody) diluted 500 to 1,000 fold with a 1 % BSA- containing PBS solution was added to the microtiterplate at a rate of 100 $\mu$l/well for incubation at 37 °C for 2 hours. For measurement of the IgM antibody titer, alkaline phosphatase-conjugated goat anti-human IgM antibody was employed. After removal of the 2ndary antibody, the microtiterplate was washed with PBST three times, and a substrate solution, i.e. an aqueous solution containing sodium p-nitrophenylphosphate (3 mg/ml) in 10 % diethanolamine buffer (pH 9.1) containing $NaN_3$ (0.2 mg/ml) and $MgCl_2.6H_2O$ (0.1 mg/ml), was added to the microtiterplate at a rate of 100 $\mu$l/well, followed by a reaction at 37 °C. The binding activity of the antibody ($OD_{405}$) was measured on a Multiskan$^R$ instrument (Titertek).

(3) Preparation of lymphocytes from human peripheral blood

Human peripheral blood (100 ml) was collected from a subject whose serum exhibited high antibody titers to P. aeruginosa Type E. 15 ml Monopoly separation liquid (Flow lab.) was charged in a centrifuge tube (Sumitomo Bakelite, 50 ml volume) and the peripheral blood (100 ml) was gently overlaid thereon. Erythrocytes and lymphocytes were separated by centrifuging with a low speed centrifuge (Tomy-Seiko BS-20BH) at 2,500 rpm (Rotor TS-7) at room temperature for 15 minutes. The layer containing lymphocytes was recovered and washed two times with RPMI 1640 medium. The number of lymphocyte cells counted was 9.5 x $10^7$.

(4) Establishment of anti-LPS antibody-producing cell by the EBV transformation method

Human peripheral lymphocytes (2 x $10^7$) were suspended in the medium (2 ml). To the suspension was added the afore-mentioned EBV solution (20 ml, virus content: $10^7$ $TD_{50}$/ml), and the mixture was incubated in the presence of 5 % $CO_2$ at 37 °C for 2 hours. EBV-infected human lymphocytes were suspended in RPMI-1640 medium to which 15 % FCS penicillin (100 IU/ml) and streptomycin (100$\mu$g/ml) had been added. The resultant suspension (about 1 x $10^5$ cells/ml) was poured into each well of a 96 well culture plate at a rate of 0.2 ml/well, to which a suspension of mouse peritoneal cells (about 1 x $10^4$ cells/well) had been added. After one week of culture, half of the culture medium was replaced by fresh medium every 3 days. On the 28th day, supernatants were separated from wells showing that cell growth appeared and analyzed by ELISA for the production of antibody reactive with the surface antigen of P. aeruginosa. EBV-transformed cells producing an IgM antibody having a potent reactivity were detected in one well.

The transformant was further cultivated and cloned by means of the limiting dilution, whereby a cell line designated HI-223, which stably produces human IgM antibody, was obtained. The term "HI-223" may also be herein used as the name of the human monoclonal antibody produced by the cell line HI-223. The human monoclonal antibody HI-223 is an IgM ($\mu$, $\lambda$).

The cell line was then subjected to the cell fusion with a mouse myeloma cell line in order to improve the proliferative property and antibody productivity.

(5) Cell fusion

HGPRT-deficient BALB/c mouse myeloma cells P3X63-Ag8.653 (ATCC No. CRL1580) which had been derived from MOPC-21 cell line were subcultured in 15 % FCS-containing Dulbecco's modified Eagle's MEM (D-MEM). The subcloned cells (1.0 x $10^7$ cells) were washed 3 times with serum-free Eagle's minimal medium . On the other hand, the EBV-transformed cell line HI-223 obtained in the above (4) (1.4 x $10^7$ cells) was washed 3 times with Eagle's minimal medium and mixed with 7 x $10^6$ of myeloma cells in a centrifuge tube (Corning #25330). Centrifugation at 400 x g for 7 minutes gave the fused cells as a precipitate. To the precipitate in the centrifuge tube, 1 ml of a polyethylene glycol (PEG) solution (0.45 g PEG 4000, Merck, 0.45 ml PBS(-) and 0.1 ml dimethyl sulfoxide) was added over about one minute while turning the tube, and then the tube was allowed to stand at room temperature for one minute. Two ml of D-MEM was added over one minute while turning the tube. This procedure was repeated four times with the same medium and three times with a 10 % FCS-containing D-MEM. Finally, 1.5 ml of FCS was added to the tube, and the mixture was left to stand for 20 minutes at 37 °C. The cells were collected by centrifugation and suspended in 30 ml of D-MEM medium containing FCS (15 %), sodium pyruvate (0.05 mg/ml), insulin (0.2 U/ml), oxaloacetic acid (0.15 mg/ml), azaserin (1 $\mu$g/ml), and hypoxanthine (100 $\mu$M), said medium being referred to as "HAz selective medium" hereinafter. The suspension was dispensed in 96 well microtiterplates (Falcon #3040) at a rate of 100 $\mu$l per plate so that one well contains 2.3 x $10^4$ myeloma cells. Each of the microplates had previously been charged with 100 $\mu$l of the suspension of feeder layer cells with the afore-mentioned medium so that each well may contain mouse BALB/c spleen cells (1 x $10^5$) and mouse BALB/c peritoneal exudate cells (1 x $10^4$), and the plates had been incubated at 37 °C for one day at 5 % $CO_2$. The microtiterplates were incubated at 37 °C at 5 % $CO_2$ and half of the culture medium was replaced by HAz selection medium at a 2 or 3 day interval. After one week, half of the culture medium was replaced by H-medium which corresponds to azaserin-free HAz selective medium. After that, the half of the medium was replaced by azaserin & hypoxanthine-free hybridoma-culturing D-MEM medium, which is D-MEM medium containing FCS (15 %), sodium pyruvate (0.05 mg/ml), insulin (0.2 U/ml), and oxaloacetic acid (0.15 mg/ml), at a 2 or 3 days interval. Production of antibody to P. aeruginosa surface antigen was determined by enzyme linked immuno sorbent assay (ELISA) with culture supernatants of the wells which showed cell growth on the 19th day after the cell fusion, by the use of 96 well microtiterplates (Falcon #3912) on which P. aeruginosa had been fixed by glutaraldehyde. P. aeruginosa PA 103 (Type E) (J. Infect Dis. 128: 506-513, 1973) (ATCC: No. 29260) was used as standard strain. A heterohybridoma was obtained from a well giving an extremely dominant cell growth and further cultivated and cloned by means of the limiting dilution, whereby a modified heterohybridoma cell line HI-223 having an improved antibody productivity and proliferative property was obtained. The thus established heterohybridoma cell line HI-223, when grown in a T-flask, showed antibody-productivity of 30 $\mu$g/ml and a doubling time of 48 hours. The cell line also grew in a serum-free medium, Cellgrosser-H, maintaining a good proliferative property and antibody productivity. The established cell line HI-223 has been deposited under an accession No. FERM P-11181 on January 9, 1990 at the Fermentation Research Institute, Agency of Industrial Science and Technology, located at Tsukuba, Ibaraki-ken, Japan and this deposition was converted into an international deposition under Budapest Treaty on December 25, 1990 under an accession No. FERM BP-3213.

Example 2

Characterization of Antigen recognized by HI-223

(1) Preparation of lipopolysaccharide (LPS) of P. aeruginosa

Lipopolysaccharides were collected from serotype standard strains of P. aeruginosa, IID1001 (Type A) and PA 103 (Type E), according to the method described by Westphal & Jann (Methods Carbohydr. Chem., 5, 83-91, 1965). Thus, wet cells (4 g) were treated with 45 % phenol at 65-68 °C, and the mixture was cooled below 10 °C and centrifuged at 3000 rpm for 15 min to separate an aqueous layer containing LPS. The aqueous solution was dialyzed against water to remove phenol and concentrated to form a micelle of LPS. The micelle, when centrifuged, gave LPS (2 mg) of P. aeruginosa Type A and E, respectively. Strains used were obtained from the Institute of Medical Science, Tokyo University, Japan. It is also available from the American Type Culture Collection (PA 103: ATCC 29260; IID1001: ATCC 27577).

(2) Characterization of antigen by Western blotting analysis

LPSs of P. aeruginosa Type A and E prepared in above (1) were separately subjected to electrophoresis on deoxycholic acid (DOC)/polyacrylamide gels according to the method described by Komuro et al. (Annual Reports of the 33rd Meeting of the Poison Symposium, p.94-99, Osaka, Japan, 1986). The gel was immersed in a transfer buffer (25 mM Tris, 192 mM glycine, pH 8.3, 20 % (v/v) methanol) at 4 °C overnight and transferred to a Durapore$^R$ filter (Millipore). Blocking was carried out by incubating the membrane sequentially in a PBS solution containing 2 % casein at room temperature for 1 hour, then in a PBS solution containing 0.1 % BSA and 10 % FCS at the same temperature for 1 hour. The blocked membrane was incubated in the supernatant of a HI-223 culture at 37 °C for 1 hour, and at 4 °C overnight. After washing with 0.05 % Tween 20-containing PBS (pH 8.0) (x5), the nitrocellulose membrane was incubated at 37 °C for 1 hour with secondary antibody which had been prepared by diluting 3,000 times peroxidase-labelled anti-human immunoglobulin antibody with PBS containing 1 % BSA and 0.05 % Tween 20. After the membrane had been washed with 0.05 % Tween 20-containing PBS (pH 8.0) 5 times, a coloring substrate (a solution of 0.5 mg/ml chloronaphtol, 20 % methanol, 0.08 % $H_2O_2$ in PBS (pH 7.5)) was added to it. A group of colored bands in a ladder-like shape were observed in a lane corresponding to the electrophoresed LPS from P. aeruginosa Type E.

Example 3

Study on Binding Spectrum of Human Monoclonal Antibody HI-223 by ELISA

(1) Binding properties of HI-223 to serotype standard strains of P. aeruginosa

The binding properties of HI-223 to the serotype standard strains of P. aeruginosa were examined by ELISA as described in Example 1-(2). The strains were obtained from the Institute of Medical Science, Tokyo University, Japan, and cultivated in heart infusion agar medium. The test results are shown in Table 1.

### Table 1  Binding Activity of HI-223 to Serotype Standard Strains of P. aeruginosa according to the Japanese Committee's Classification

| Serotype | Strain | ELISA Value ($OD_{405}$) |
|---|---|---|
| A | IID 1001 (ATCC27577) | 0.18 |
| B | IID 1002 (ATCC27578) | 0.14 |
| B | IID 1007 (ATCC27583) | 0.13 |
| B | IID 1013 (ATCC27589) | 0.13 |
| B | IID 5004 | 0.12 |
| C | IID 1021 | 0.14 |
| D | IID 1004 (ATCC27580) | 0.16 |
| E | IID 1130 | 2.56 |
| F | IID 1006 (ATCC27582) | 0.14 |
| G | IID 1020 | 0.14 |
| H | IID 1009 (ATCC27585) | 0.11 |
| I | IID 1010 (ATCC27586) | 0.08 |
| J | IID 1011 (ATCC27587) | 0.09 |
| K | IID 1012 (ATCC27588) | 0.07 |
| L | IID 5141 | 0.10 |
| M | IID 5018 | 0.12 |
| M | IID 1015 | 0.08 |

The table shows that HI-223 bound selectively to the serotype standard strains of P. aeruginosa Type E.

(2) Binding properties of HI-223 to clinical isolates

It was shown in the above test that HI-223 can bind to the serotype standard strains of Type E. The binding properties of HI-223 to 20 clinical isolates of the serotype of Type E was then examined. The results are shown in Table 2. As can be seen from the Table 2, HI-223 reacted with all isolates. About each 20 strains of other serotypes which are clinically isolated with high frequency, such as Type A, B, G, I and M, were also examined for the antibody-binding activity. HI-223 did not bind to any of these strains.

## Table 2  Binding Activity of HI-223 to Clinical Isolates of serotype E

| Strain | ELISA Value ($OD_{405}$) |
|---|---|
| SP9702 | 2.35 |
| SP9715 | 2.41 |
| SP9720 | 2.28 |
| SP9723 | 2.27 |
| SP9726 | 2.40 |
| SP9733 | 2.37 |
| SP9740 | 2.37 |
| SP9754 | 2.26 |
| SP9759 | 2.37 |
| SP9771 | 2.36 |
| SP9783 | 2.36 |
| SP9787 | 2.32 |
| SP9790 | 2.33 |
| SP10043 | 2.30 |
| SP10059 | 2.34 |
| PA103 | 2.32 |
| PA103-29 | 2.33 |
| NC5 | 2.30 |
| TL2092 | 2.06 |
| TL2158 | 2.27 |
| negative control (-) | 0.00 |

The results obtained in Examples 2 and 3 demonstrate that human monoclonal antibody HI-223 recognizes O-antigen (serotype-specific antigen) in the LPS of P. aeruginosa Type E.

Example 4

Jar fermenter culture of the hybridoma HI-223

A large scale culture of the hybridoma HI-223 was grown using a jar fermenter (volume, 6 l; Model MCT-3S, Marubishi Bioeng., Japan). The culture medium used was commercially available serum-free Cellgrosser H medium (Meguro Institute, Japan) supplemented with glucose (to a final concentration of 4 mg/ml). The culture medium (4 l) was innoculated with hybridoma cells ($0.35 \times 10^5$ cells/ml). After 3 days of cultivation, the cell concentraton had increased up to $4.8 \times 10^5$ cells/ml and the human monoclonal antibody IgM present in the supernatant was 11.8 $\mu$g/ ml. When a continuous culture was conducted employing the gravity sedimentation method, the cell concentration was maintained above 1 x 10$^6$ cells/ml while the antibody concentration in the culture supernatant was up to 23.5 $\mu$g/ml.

Example 5

Purification of specific antibody

The culture supernatant (47 l) obtained from a large-scale culture which was conducted in the same manner as in Example 3 using serum-free Cellgrosser H medium in a Jar fermentor (6 l volume) was concentrated to 1.75 l by ultracondensation. The concentrated supernatant was charged in a Q-sepharose FF column (Pharmacia; 2.6 cm $\emptyset$ x 30 cm; volume: 150 ml) previously equilibrated with 0.1 M phosphate buffer (pH 6.8). The column was washed with about 5 volumes of 0.1 M phosphate buffer (pH 6.8) and eluted with 0.5 M phosphate buffer to obtain fractions containing IgM. Fractions were subjected to the ultra-condensation and charged in a gel filtration column, Sephacryl™ S 300 HR (Pharmacia; 2.6 cm $\emptyset$ x 90 cm; volume: 450 ml). The column was eluted with PBS (-). Fractions were assayed by ELISA for the antibody titer of anti-P. aeruginosa (Type E) antibody. From the fractions containing antibody activity 500 mg of human monoclonal IgM antibody were recovered.

Example 6

Therapeutic effect of human monoclonal antibody HI-223 on experimental infection of P. aeruginosa in mouse

(1) Effect of monoclonal antibody administration alone

Two standard strains and 3 clinically isolated strains of P. aeruginosa type E were used in the test for the evaluation of the therapeutic effect of HI-223. ICR-scl mice (4 week old, male, 10 animals per group) were administered intraperitoneally with a 5 % mucin-containing suspension of P. aeruginosa. One hour later, each mouse was injected intraperitoneally with 0.1 $\mu$g/head of human monoclonal antibody HI-223. The terapeutic effect was determined on the basis of the survival ratio on the 6th day. The results are shown in Table 3.

Table 3  Therapeutic effect of HI-223 on experimental

P. aeruginosa infection in mouse


Therapeutic effect of monoclonal antibody
administration alone

The survival ratios on the 6th day in a group
consisting of 10 mice

Dose of HI-223: 0.1 µg/head

| Strain | PA103 | Survival ratio (%) | | |
|---|---|---|---|---|
| | Inoculation rate of bacteria (CFU/head) | | | |
| | $4.0 \times 10^3$ | $2.0 \times 10^4$ | $1.0 \times 10^5$ | $4.0 \times 10^5$ | $2.0 \times 10^6$ |
| HI-223 | N.D.* | 100 | 90 | 60 | 30 |
| non treated | 100 | 20 | 20 | 0 | N.D. |

| Strain | NC5 | Survival ratio (%) | | |
|---|---|---|---|---|
| | Inoculation rate of bacteria (CFU/head) | | | |
| | $2.3 \times 10^4$ | $1.1 \times 10^5$ | $4.5 \times 10^5$ | $2.3 \times 10^6$ | $1.1 \times 10^7$ |
| HI-223 | N.D. | 90 | 90 | 0 | 0 |
| non treated | 70 | 60 | 30 | 0 | N.D. |

| Strain | SP9771 | | Survival ratio (%) | |
|---|---|---|---|---|
| | Inoculation rate of bacteria (CFU/head) | | | |
| | $1.9 \times 10^6$ | $7.4 \times 10^6$ | $3.7 \times 10^7$ | $1.9 \times 10^8$ |
| HI-223 | 90 | 90 | 10 | 0 |
| non treated | 40 | 10 | 0 | 0 |

| Strain | SP9783 | | Survival ratio (%) | | |
|---|---|---|---|---|---|
| | Inoculation rate of bacteria (CFU/head) | | | | |
| | $3.6 \times 10^3$ | $1.8 \times 10^4$ | $8.9 \times 10^4$ | $3.6 \times 10^5$ | $1.8 \times 10^6$ |
| HI-223 | N.D. | 100 | 100 | 100 | 50 |
| non treated | 100 | 90 | 50 | 30 | N.D. |

| Strain | SP10043 | | Survival ratio (%) | | | |
|---|---|---|---|---|---|---|
| | Inoculation rate of bacteria (CFU/head) | | | | | |
| | $9.3 \times 10^2$ | $3.7 \times 10^3$ | $1.9 \times 10^4$ | $9.3 \times 10^4$ | $3.7 \times 10^5$ | $1.9 \times 10^6$ |
| HI-223 | N.D. | N.D. | 100 | 100 | 100 | 70 |
| non treated | 100 | 80 | 30 | 20 | N.D. | N.D. |

N.D.: Not tested

The $LD_{50}$ value (the inoculation rate of bacteria which brings 50 % of mice to death) and the range (difference) between the upper limit and the lower limit of 95 % confidence limits were calculated statistically from the data shown in Table 3. The results are shown in Table 4. As can be seen from Table 4, the $LD_{50}$ value is higher in the treated group than in the control group (non-treated group). As to the range between the upper limit and the lower limit of confidence limits, there are no overlapping ranges between the two groups and the difference is significant. These results obviously demonstrate that HI-223 IgM is therapeutically effective on the P. aeruginosa infections at the administration rate of 0.1 μg/head.

Table 4  Comparison of $LD_{50}$ value

Strain        PA103

| | $LD_{50}$ value (95 % Confidence limits) |
|---|---|
| HI-223 | $7.5 \times 10^5$ ($2.8 \times 10^5 - 2.0 \times 10^6$) |
| non-treated | $1.9 \times 10^4$ ($6.0 \times 10^3 - 5.8 \times 10^4$) |

Strain        NC5

| | $LD_{50}$ value (95 % Confidence limits) |
|---|---|
| HI-223 | $7.2 \times 10^5$ ($3.8 \times 10^5 - 1.4 \times 10^6$) |
| non-treated | $1.1 \times 10^5$ ($4.0 \times 10^4 - 2.7 \times 10^5$) |

Strain        SP9771

| | $LD_{50}$ value (95 % Confidence limits) |
|---|---|
| HI-223 | $1.4 \times 10^7$ ($7.2 \times 10^6 - 2.6 \times 10^7$) |
| non-treated | $1.4 \times 10^6$ ($4.8 \times 10^5 - 4.1 \times 10^6$) |

Strain        SP9783

| | $LD_{50}$ value (95 % Confidence limits) |
|---|---|
| HI-223 | $1.0 \times 10^6$ ($9.7 \times 10^5 - 3.3 \times 10^6$) |
| non-treated | $1.2 \times 10^5$ ($4.7 \times 10^4 - 3.2 \times 10^5$) |

Strain        SP10043

| | $LD_{50}$ value (95 % Confidence limits) |
|---|---|
| HI-223 | $2.9 \times 10^6$ ($1.4 \times 10^6 - 6.4 \times 10^6$) |
| non-treated | $1.5 \times 10^4$ ($5.6 \times 10^3 - 3.9 \times 10^4$) |

(2) Effect of combined treatment with various antibiotics

Therapeutic effects of combined treatments of human monoclonal antibody HI-223 and various antibiotics on experimentally infected mice was evaluated using the P. aeruginosa strain PA-103, a standard strain of P. aeruginosa Type E, as infectious pathogen substancially in accordance with the procedure described in the above (1).

Experimentally infected mice (8 mice per group, amount of inoculated bacteria; $6.5 \times 10^5$ CFU/head) were treated with antibiotics (subcutaneous injection) and/or HI-223 (intraperitoneal injection) 1 and 4 hours after inoculation of the pathogen in different ways as shown below in Table 5. The evaluation of therapeutic effects was carried out on the basis of the survival ratio of each group after 1 week from the treatment.

The dose of agents was determined as follows so that the survival ratio may be about 10 % when it is administered alone: HI-223 IgM : 0.1 $\mu$g/head ; imipenem cilastatin mixture (IPM/CS, Tienam[R], Banyu, Japan) : 0.02 mg/head; meropenem (SM-7338, Sumitomo, Japan) : 0.07 mg/head; tobramycin (TOB, Tobracin[R], Shionogi, Japan) : 0.03 mg/head; gentamicin (GM, Gentacin[R], Essex Japan, Japan) : 0.2 mg/head; ceftazidime (CAZ, Modacin[R], Tanabe, Japan) : 1 mg/head; cefsulodin (CFS, Takesulin[R], Takeda, Japan) : 2 mg/head; cefpiramide (CPM, Sepatren[R], Sumitomo, Japan) : 5 mg/head; aztreonam (AZT, Azactam[R], Ezai, Japan) : 6 mg/head; carumonam (CRMN, Amasulin[R], Takeda, Japan) : 4mg/head; piperacillin (PIPC, Pentcilin[R], Toyama, Japan) : 50mg/head.

The results are shown in Table 5, which demonstrate that HI-223 shows a potent synergistic effect when used together with carbapenem or aminoglycoside antibiotics. On the contrary, it shows only an additive effect when used together with cephalosporin, monobactam and penicillin antibiotics.

### Table 5  Effect of combined treatment of HI-223 with various antibiotics

The survival ratio on the 7th day in a group consisting of 8 mice

(P. aeruginosa  strain PA 103  )

| | administration after infection | | survival ratio |
|---|---|---|---|
| | 1 hour | 4 hours | (%) |
| carbapenems | IPM/CS | -- | 12.5 |
| | IPM/CS | HI-223 | 62.5 |
| | SM-7338 | -- | 0 |
| | SM-7338 | HI-223 | 87.5 |
| amino-glycosides | TOB | -- | 0 |
| | TOB | HI-223 | 75 |
| | GM | -- | 12.5 |
| | GM | HI-223 | 100 |
| cephalo-sporins | CAZ | -- | 0 |
| | CAZ | HI-223 | 25 |
| | CFS | -- | 0 |
| | CFS | HI-223 | 25 |
| | CPM | -- | 12.5 |
| | CPM | HI-223 | 25 |
| mono-bactams | AZT | -- | 12.5 |
| | AZT | HI-223 | 25 |
| | CRMN | -- | 0 |
| | CRMN | HI-223 | 37.5 |
| penicillins | PIPC | -- | 0 |
| | PIPC | HI-223 | 12.5 |
| | HI-223 | -- | 25 |
| | HI-223 | HI-223 | 12.5 |

(3) Influence of the time of administration of antibody HI-223 in the combined treatment

The influence of the time of administration of HI-223 and the antibotic on the therapeutic effects was evaluated using the imipenem antibiotic which was demonstrated to be highly effective in the combined treatment as can be seen in Table 5.

The infection model system obtained in the same manner as (1) was used in this experiment. Each animal received subcutaneous injection with IPM/CS (0.5 mg/head) 1 hour after the infection. Antibody treatment was conducted by single intraperitoneal injection of HI-223 (0.1 μg/head) at 1, 2, 4, 6 or 8 hours after the infection. The therapeutic effect was evaluated on the basis of the survival ratios after 1 week. The combined treatment appeared to be more effective when the antibody is administered 4 to 8 hours after infection than is administered 1 hour after infection (together with antibiotic), although the difference cannot be regarded as significant (Table 6).

<u>Table 6</u>  Influence of the administration time of HI-223

in the combined treatment with imipenem (1)

The survival ratios on the 7th day in a group
consisting of 5 mice

Administration of IPM/CS: 1 hour after infection

| Strain          PA103 | Survival ratio (%) | |
|---|---|---|
| Administration time of HI-223 (after infection) | Amount of inoculated bacteria (CFU/head) | |
| | $1.1 \times 10^7$ | $5.4 \times 10^7$ |
| 1 hour | 100 | 0 |
| 2 hours | 100 | 0 |
| 4 hours | 100 | 40 |
| 6 hours | 100 | 20 |
| 8 hours | 100 | 40 |
| -- * | 20 | 20 |

*:  single administration of IPM/CS

Further experiments were carried out to compare the effect of administration time of HI-223, i.,e., 1 and 4 hours after infection. Thus, in the infection model system (10 mice per group) described in (1), animals received either of the following injections 1 or 4 hours after infection: subcutaneous injection of IPM/CS (0.5 mg/head) or intraperitoneal injection of HI-223 (0.1 μg/head). The results are shown in Table 7.

Table 7   Influence of the administration time of HI-223 in the combined treatment with imipenem (2)

The survival ratios on the 7th day in a group consisting of 10 mice

| Strain PA103 | | Survival ratio (%) | | | | |
|---|---|---|---|---|---|---|
| Treatment time after infection | | Amount of inoculated bacteria (CFU/head) | | | | |
| 1 hour | 4 hours | $2.8 \times 10^5$ | $1.4 \times 10^6$ | $5.6 \times 10^6$ | $2.8 \times 10^7$ | $1.4 \times 10^8$ |
| IPM/CS | IPM/CS | N.D. * | N.D. | 70 | 30 | 10 |
| IPM/CS | HI-223 | N.D. | N.D. | 90 | 60 | 10 |
| HI-223 | HI-223 | 50 | 30 | 10 | 10 | N.D. |
| IPM/CS | IPM/CS HI-223 | N.D. | N.D. | 100 | 60 | 50 |
| IPM/CS HI-223 | -- ** | N.D. | N.D. | 70 | 10 | 40 |
| IPM | -- | N.D. | 40 | 10 | 0 | N.D. |
| HI-223 | -- | 20 | 10 | 10 | 0 | N.D. |

| survival ratio of non treated group | $2.8 \times 10^3$ | $1.4 \times 10^4$ | $5.6 \times 10^4$ | $2.8 \times 10^5$ |
|---|---|---|---|---|
| | 80 | 30 | 20 | 0 |

*   N.D.:   not tested

** --:   not administered

The LD$_{50}$ value and the range (difference) between the upper limit and the lower limit of 95 % confidence limits were calculated statistically from the data shown in Table 7. The results are shown in Table 8.

### Table 8  Comparison of $LD_{50}$ values

| Treatment after infection | | $LD_{50}$ (95 % confidence limits) |
|---|---|---|
| 1 hour | 4 hours | |
| IPM/CS | IPM/CS | $1.3 \times 10^7$ $(4.4 \times 10^6 - 3.8 \times 10^7)$ |
| IPM/CS | HI-223 | $3.3 \times 10^7$ $(1.1 \times 10^7 - 9.7 \times 10^7)$ |
| HI-223 | HI-223 | $2.4 \times 10^5$ $(4.9 \times 10^4 - 1.2 \times 10^6)$ |
| IPM/CS | IPM/CS HI-223 | $9.7 \times 10^7$ $(3.1 \times 10^7 - 3.0 \times 10^8)$ |
| IPM/CS HI-223 | -- * | $9.5 \times 10^6$ $(1.2 \times 10^6 - 7.7 \times 10^7)$ |
| IPM | -- | $1.1 \times 10^6$ $(3.6 \times 10^5 - 3.1 \times 10^6)$ |
| HI-223 | -- | $< 2.8 \times 10^5$ |
| not administered | | $9.1 \times 10^3$ $(3.4 \times 10^3 - 2.4 \times 10^4)$ |

\* --:  not administered

As can be seen from Table 8, the $LD_{50}$ value is higher in groups corresponding to the combined treatment than groups corresponding to the single administration with IPM/CS or HI-223. Concerning to the differences (provided in parenthesis) between the upper and the lower limits of 95 % confidence limits, there is no overlapping range between combined-treatment groups and single-administration groups and the difference is significant.

In analogy to the previous test, in the groups of combined treatment, the group which was treated by administrating HI-223 4 hours after infection showed higher a $LD_{50}$ value (without significancy) than that which was treated by administrating HI-223 1 hour after infection, suggesting that the effect of combined treatment can be improved by delaying the antibody therapy for a certain period.

Although IPM/CS was more effective than HI-223 in case of a single administration, the $LD_{50}$ value of the group treated by administrating IPM/CS twice (1 and 4 hours after infection) was lower than that of the group treated by a combined treatment (IPM/CS: 1 hour after infection; HI-223 : 4 hours after infection) (no significance exists). It is notable that the therapeutic effect was further improved by administrating IPM/CS twice at 1 and 4 hours after infection and HI-223 once at 4 hours after infection.

Example 7

Evaluation of opsonization activity of monoclonal antibody HI-223

The following experiments were conducted simultaneously.

(1) Opsonization activity of HI-223

1) Preparation of mouse resident peritoneal exudate cells (PEC).

Mouse resident peritoneal exudate cells (PEC) were prepared by subjecting mice (SLC, 5 weeks, male) to exsanguination, followed by a peritoneal injection with a mixture of 5 U/ml of heparin-0.1% bovin serum albumin (BSA) solution-Hank's solution (all are available from Wako Junyaku, Japan). The following procedures were carried out under ice-cooling. The resultant PEC was then washed twice with Hank's solution and diluted with 25 mM HEPES (Wako Junyaku, Japan)-$RPMI_{1640}$ (ibid.) to $2 \times 10^6$ cells/ml. More than 95 % of the cells were macrophages.

22

2) Absorption of normal mouse serum (NMS) by living bacteria

A suspension of P. aeruginosa strain PA-103 bacteria in (PBS-) was added to NMS (about $2\times10^9$ CFU/ml) and the mixture was allowed to stand at 4 °C for 1 hour and centrifuged to collect the supernatant. These procedures were repeated twice. The resultant serum was served as absorbed serum (A-NMS) after filter sterilization using a 0.22 $\mu$ filter.

3) Inactivation of serum

The A-NMS was allowed to stand for about 30 minutes at 56°C to inactivate the complement and used as inactivated serum (I-NMS).

4) Preparation of bacteria

A suspension (about $1 \times 10^9$ CFU/ml) of P. aeruginosa strain PA-103 bacteria in (PBS-) was diluted 1000 times with 25 mM HEPES-RPMI$_{1640}$ to obtain a bacterial solution.

5) Measurement of the phagocytotic activity of peritoneal macrophages

In this measurement, PEC solution (macrophage), serum (A-NMS or I-NMS), antibody (HI-223) and bacterial solution (P. aeruginosa) were used. A mixture of PEC solution (0.5 ml), serum (A-NMS, 0.2 ml), antibody (HI-223 (0.5 $\mu$g/ml), 0.2 ml) and bacterial solution (0.1 ml) was charged into a Petri-dish for cell culture (35 mm $\emptyset$, Corning). The dish was placed in another Petri-dish (90 mm $\emptyset$, Termo) to prevent drying and incubated on gyrotory shaker at 100 rpm. After 2 hours incubation at 37 °C, viable bacteria were counted. A control experiment was carried out using the same mixture without HI-223.

6) Results

The influence of HI-223 on phagocytotic activity of peritoneal macropharges was estimated and shown in Table 9. It can be seen from the table that the amount of the viable bacteria in the reaction system comprising HI-223 is significantly decreased compared to those in the control system which lacks HI-223, suggesting that said antibody has an opsonization activity.

(2) Opsonization of HI-223 in the presence of imipenem

The opsonization activity of HI-223 in the presence of 2, 1/2, or 1/8 MIC of imipenem cilastatin sodium (IPM/CS) was evaluated in the same manner as above except that IPM/CS was added to the reaction systems described in (1) above to the final concentrations of 0.8, 0.2, or 0.05 $\mu$g/ml. The results are shown in Table 10. As can be seen from the table, in reaction systems which lack HI-223, the number of viable bacteria was decreased to 3.4% and 27% in the presence of 2 and 1/2 MIC of IPM/CS, respectively, while it was not changed in the presence of 1/8 MIC of IPM/CS. It can be also observed that when HI-223 was added to the above reaction system the number of viable bacteria was further decreased (18% and 30%). The extent of decrease is consistent with that obtained in the IPM/CS-free reaction system (see Table 9). As a conclusion, the effect of HI-223 and IPM/CS is additive. HI-223 has an opsonization activity by which it activates phagocytosis of macrophages, whereas IPM/CS has bacteriolytic activity.

Table 9  Opsonization activity of HI-223

| Reaction system | | Final cell count (CFU/ml) | Comparison with control |
|---|---|---|---|
| PEC, | A-NMS | $1.75 \times 10^5$ | 100 |
| PEC, HI-223, | A-NMS | $3.08 \times 10^4$ | 18 |
| PEC, | I-NMS | $1.50 \times 10^5$ | 86 |

control: PEC, A-NMS

Table 10  Opsonization activity of HI-223 in the presence of imipenem

IPM/CS    0.8 µg/ml (2MIC)

| Reaction system | | Final cell count (CFU/ml) | Comparison with control |
|---|---|---|---|
| PEC, IPM, | A-NMS | $5.90 \times 10^3$ | 100 |
| PEC, IPM, HI-223, | A-NMS | $1.78 \times 10^3$ | 30 |
| PEC, IPM, | A-NMS | $6.18 \times 10^3$ | 109 |

IPM/CS    0.2 µg/ml (1/2MIC)

| Reaction system | | Final cell count (CFU/ml) | Comparison with control |
|---|---|---|---|
| PEC, IPM, | A-NMS | $4.73 \times 10^4$ | 100 |
| PEC, IPM, HI-223, | A-NMS | $8.65 \times 10^3$ | 18 |
| IPM, | A-NMS | $6.10 \times 10^4$ | 129 |

IPM/CS    0.05 µg/ml (1/8MIC)

| Reaction system | | Final cell count (CFU/ml) | Comparison with control |
|---|---|---|---|
| PEC, IPM, | A-NMS | $1.64 \times 10^5$ | 100 |
| PEC, IPM, HI-223, | A-NMS | $2.91 \times 10^4$ | 18 |
| IPM, | A-NMS | $1.42 \times 10^5$ | 87 |

control: PEC, IPM (each dose), A-NMS

Example 8

Therapeutic effects of human monoclonal antibody MH-4H7 in association with various antibiotics on experimental infection of P. aeruginosa in mouse

(1) Effect of combined treatment with various antibiotics

In the present experiment, an antibody specific for the outer core of P. aeruginosa is used. The antibody MH-4H7 was previously established and disclosed in the Japanese Patent Publication (KOKAI) No. 84197/1990.

An experimental-mouse-infection system was prepared in accordance with the procedures described in Example 6 - (1) using a clinically isolated P. aeruginosa SP-6788 (Type G).

Experimentally infected mice (10 mice per group) were injected intraperitoneally with MH-4H7 and either of the following antibiotics 1 hour after infection. The therapeutic effect was evaluated on the basis of the survival ratios on the 6th day.

MH-4H7IgM : 0.05 $\mu$g/head ; imipenem cilastatin mixture (IPM/CS, Tienam[R], Banyu, Japan) : 0.1 mg/head; tobramycin (TOB, Tobracin[R], Shionogi, Japan) : 0.05 mg/head; ceftazidime (CAZ, Modacin[R], Tanabe, Japan): 0.5 mg/head.

The results are shown in Table 11, which demonstrate that MH-4H7 shows a synergistic effect when used together with imipenem cilastatin mixture (carbapenem antibiotic) and tobramycin (aminoglycoside antibiotic), while it shows only an additive effect when used together with ceftazidime (cephalosporin anitibiotic).

Table 11  Effect of combined treatment of MH-4H7 with various antibiotics

The survival ratio on the 6th day in a group consisting of 10 mice

(P. aeruginosa strain SP-6788  )

| In association with IPM | Inoculation rate of bacteria | $7.0 \times 10^5$ CFU/head |
|---|---|---|
| **Agents administered** | **survival ratio (%)** | |
| IPM/CS | 40 | |
| MH-4H7 | 20 | |
| IPM/CS+MH-4H7 | 100 | |
| non-treated | 20 | |

| In association with TOB | Inoculation rate of bacteria | $2.0 \times 10^6$ CFU/head |
|---|---|---|
| TOB | 30 | |
| MH-4H7 | 10 | |
| TOB+MH-4H7 | 80 | |
| non-treated | 20 | |

| In association with CAZ | Inoculation rate of bacteria | $8.2 \times 10^5$ CFU/head |
|---|---|---|
| CAZ | 0 | |
| MH-4H7 | 50 | |
| CAZ+MH-4H7 | 70 | |
| non-treated | 0 | |

(2) Influence of the time of administration of antibody MH-4H7 in the combined treatment with imipenem

The influence of the time of administration of MH-4H7 on the therapeutic effects of combined treatment with antibiotics was investigated using clinically isolated P. aeruginosa SP-6788 (Type G). Imipenem which was demonstrated to be highly effective in such a treatment was used as an antibiotic.

The infection model system obtained in the same manner as in Example 6-(1) was used in this experiment. Each animal received subcutaneous injection with IPM/CS (0.5 mg/head) 1 hour after the infection. Treatment with antibody was conducted by a single intraperitoneal injection of MH-4H7 (0.1 μg/head) at 1, 2, 4, 6 or 8 hours after the infection. The therapeutic effect was evaluated on the basis of the survival ratios after 1 week. The results are shown in Table 12. The combined treatment appeared to be more effective when the antibody is administered 4 to 8 hours after infection than is administered 1 hour after infection (simultaneously, together with antibody), although the difference cannot be regarded as significant.

Table 12  Influence of the administration time of

MH-4H7 in the combined treatment with imipenem (1)

The survival ratios on the 7th day in a group
consisting of 5 mice

Administration of IPM/CS: 1 hour after infection

Strain:      SP-6788:          Survival ratio (%)

| Administration time of MH-4H7 (after infection) | Amount of Inoculated of bacteria (CFU/head) 6.6 x 10$^6$ |
|---|---|
| 1 hour | 60 |
| 2 hours | 20 |
| 4 hours | 80 |
| 6 hours | 100 |
| 8 hours | 100 |
| -- * | 0 |

*:  single administration of IPM/CS

A further experiment was carried out to compare the effect of administration time of MH-4H7, i.e., 1 and 6 hours after infection. Thus, in the infection model system (10 mice per group) described in Example 6-(1), animals were treated by either of the following injections at the time of 1 or 6 hours after infection: subcutaneous injection of IPM/CS (0.5 mg/head) or intraperitoneal injection of MH-4H7 (1 µg/head). The results are shown in Table 13.

Table 13  Influence of the administration time of MH-4H7 in the combined treatment with imipenem (2)

The survival ratios on the 7th day in a group consisting of 10 mice

| Strain: | SP-6788: | | | Survival ratio (%) | |
|---|---|---|---|---|---|
| Treatment time after infection | | Amount of Inoculated bacteria (CFU/head) | | | |
| 1 hour | 6 hours | $3.2 \times 10^5$ | $1.3 \times 10^6$ | $6.3 \times 10^6$ | $3.2 \times 10^7$ |
| IPM/CS | IPM/CS | N.D. * | 100 | 20 | 10 |
| IPM/CS | MH-4H7 | N.D. | 90 | 80 | 20 |
| MH-4H7 | MH-4H7 | 100 | 70 | 20 | N.D. |
| IPM/CS MH-4H7 | -- ** | 100 | 100 | 40 | 0 |
| IPM/CS | -- | 100 | 70 | 20 | N.D. |
| MH-4H7 | -- | 100 | 50 | 10 | N.D. |

* N.D.: not tested

** --: not administered

| Non treated group | Survival ratio (%) | | |
|---|---|---|---|
| Amount of inoculated bacteria (CFU/head) | | | |
| $6.3 \times 10^2$ | $3.2 \times 10^3$ | $1.3 \times 10^4$ | $6.4 \times 10^4$ |
| 100 | 90 | 10 | 10 |

The $LD_{50}$ value and the range (difference) between the upper limit and the lower limit of 95 % confidence limits were calculated statistically from the data shown in Table 13. The results are shown in Table 14.

### Table 14 Comparison of $LD_{50}$ values

| Treatment after infection | | $LD_{50}$ (CFU/head) |
| --- | --- | --- |
| 1 hour | 6 hours | (95 % confidence limits) |
| IPM/CS | IPM/CS | $5.0 \times 10^6$ ($2.1 \times 10^6$ – $1.2 \times 10^7$) |
| IPM/CS | MH-4H7 | $1.2 \times 10^7$ ($5.1 \times 10^6$ – $3.0 \times 10^7$) |
| MH-4H7 | MH-4H7 | $2.5 \times 10^6$ ($1.4 \times 10^6$ – $4.6 \times 10^6$) |
| IPM MH-4H7 | -- * | $6.0 \times 10^6$ ($3.3 \times 10^6$ – $1.1 \times 10^7$) |
| IPM/CS | -- | $2.5 \times 10^6$ ($1.4 \times 10^6$ – $4.6 \times 10^6$) |
| MH-4H7 | -- | $1.6 \times 10^6$ ($7.2 \times 10^5$ – $3.5 \times 10^6$) |
| not administered | | $7.8 \times 10^3$ ($4.1 \times 10^3$ – $1.5 \times 10^4$) |

\* --: not administered

As can be seen from Table 14, the $LD_{50}$ value is higher in groups corresponding to the combined treatment than in groups corresponding to the single administration with IPM/CS or MH-4H7. Concerning the differences (provided in parenthesis) between the upper and the lower limits of 95 % confidence limits, there is no overlapping range between combined-treatment groups and single-administration group and the difference is significant.

In analogy to the previous test with the groups of combined treatment, the group which was treated by administering MH-4H7 6 hours after infection showed a higher $LD_{50}$ value (without significancy) than that treated by administering MH-4H7 1 hour after infection, suggesting that the effect of combined treatment can be improved by delaying the antibody therapy for a certain period. These results were consistent with those obtained in the previous Example 6 using human monoclonal antibody HI-223.

Example 9

Therapeutic effects of human monoclonal antibody IN-2A8 in association with an antibiotic imipenem on experimental infection of P. aeruginosa in mouse

In the present experiment, an antibody specific for the flagellum of P. aeruginosa is used. The antibody IN-2A8 was previously established and disclosed in the Japanese Patent publication (KOKAI) No. 299594/1990.

An experimental mouse infection system was prepared in accordance with the procedures described in Example 6 - (1) using a clinically isolated P. aeruginosa PS-10052 (Type B).

Experimentally infected mice (10 mice per group) were injected intraperitoneally with IN-2A8 (1 μg/head) and an imipenem cilastatin mixture (0.1 mg/head). The therapeutic effect was evaluated on the basis of the survival ratios on the 6th day. Results are shown in Table 15.

Table 15   Effect of combined treatment of IN-2A8 with imipenem

The survival ratio on the 6th day in a group consisting of 10 mice

(P. aeruginosa strain SP-10052   )

Survival ratios

| Administered agents | Amount of Inoculated bacteria (CFU/head) | | | |
|---|---|---|---|---|
| | $3.0 \times 10^6$ | $1.5 \times 10^7$ | $6.1 \times 10^7$ | $3.0 \times 10^8$ |
| IPM/CS | 100 | 20 | 0 | 10 |
| IN-2A8 | 30 | 20 | 10 | 10 |
| IPM/CS+ IN-2A8 | 100 | 100 | 40 | 30 |

| Non treated group | Survival ratio (%) | | |
|---|---|---|---|
| Amount of inoculated bacteria (CFU/head) | | | |
| $3.0 \times 10^4$ | $3.0 \times 10^5$ | $3.0 \times 10^6$ | $1.5 \times 10^7$ |
| 70 | 30 | 10 | 0 |

The $LD_{50}$ value and the range (difference) between the upper limit and the lower limit of the 95 % confidence limits were calculated statistically from the data shown in Table 15. The results are shown in Table 16.

Table 16   Comparison of $LD_{50}$ values

| | $LD_{50}$ (95 % confidence limits) |
|---|---|
| IPM/CS | $1.1 \times 10^7$ ($4.9 \times 10^6 - 2.6 \times 10^7$) |
| IN-2A8 | $< 3.0 \times 10^6$ |
| IPM/CS+ IN-2A8 | $9.6 \times 10^7$ ($4.2 \times 10^7 - 2.2 \times 10^8$) |
| not administered | $6.6 \times 10^4$ ($7.3 \times 10^3 - 6.0 \times 10^5$) |

As can be seen from Table 16, the $LD_{50}$ value is higher in groups corresponding to the combined treatment than groups corresponding to the single administration with IPM/CS or IN-2A8. Concerning the differences (provided in parenthesis) between the upper and the lower limits of the 95 % confidence limits, there is no overlapping range between combined-treatment and single-administration groups and the difference is significant. These results are consistent with those obtained using human monoclonal antibody HI-223 or MH-4H7.

Example 10

Effects of HI-223 on the mouse-endotoxin shock model

The present experiment was carried out to determine whether antibody HI-223 specifically reactive with the O-antigen of P. aeruginosa possesses in vivo neutralizing activity against LPS. The experiment was conducted using a mouse-endotoxin shock model substancially in accordance with Galactosamine Sensitizing method (Proc.Acad.Sci. USA 76: 5939-5943 (1979)), because the mouse is relatively tolerant to the lethal effect of endotoxin. Endotoxin shocks were induced in BALB/c mice (6 weeks, female, 10 animals per group) by peritoneal injection with a mixture containing LPS. The mixture for injection was prepared by dissolving LPS from P. aeruginosa strain PA 103 bacteria in a physiological saline (0.2 ml) and adding D-galactosamine (15 mg) thereto. Each animal received an injection with HI-223 (10 $\mu$g/head) either intravenously (1 hour before the induction of the shock) or intraperitoneally (5 minutes after the induction). A control experiment was carried out by injecting a mixture containing bovine serum albumin (BSA) instead of HI-223, at the same concentration. After 1 week of observation for deaths, the median lethal dose ($LD_{50}$) was calculated. Results are shown in Tables 17 and 18. Table 17 shows the results obtained by administering HI-223 1 hour before induction of the shock, while Table 18 shows those obtained by administering it after 5 minutes from the induction. These tables show that the survival ratios in HI-223-treated groups are higher than those of the non-treated control group and the difference is significant. It demonstrates that monoclonal antibody HI-223 is therapeutically effective in the treatment and prophylaxis of endotoxin shock.

Table 17   Endotoxin shock-preventive effect of HI-223

| Treatment (- 1h, i.p.) | dose ($\mu$g/head) | $LD_{50}$ of LPS (ng/head) | Comparison with control |
|---|---|---|---|
| HI-223 | 10 | 426 | 30 |
| BSA | 10 | 14 | 1.0 |

p< 0.05

Table 18   Endotoxin shock-therapeutic effect of HI-223

| Treatment (5 min, i.v.) | dose ($\mu$g/head) | $LD_{50}$ of LPS (ng/head) | Comparison with control |
|---|---|---|---|
| HI-223 | 10 | 898 | 13 |
| BSA | 10 | 68 | 1.0 |

p< 0.05

Claims

1. A human monoclonal antibody which is obtainable from hybridoma cell line HI-223, which has been deposited with the Fermentation Research Institute, Agency of Industrial Science and Technology under the provisions of the Budapest Treaty under deposit accession number FERM BP-3213, or its descendant cell line, said human monoclonal antibody having the following characteristics:

   1) the antigenic determinant for the monoclonal antibody is an O-antigen of P. aeruginosa;

2) the monoclonal antibody is prophylactically and therapeutically effective on infections caused by P. aeruginosa;

3) the monoclonal antibody shows synergistic effects in the treatment of experimental P. aeruginosa infections when used in association with carbapenem or aminoglycoside antibiotics;

4) the monoclonal antibody belongs to the IgM (Immunoglobulin M) class; and

5) the human monoclonal antibody recognizes lipopolysaccharides of P. aeruginosa strains of serotype E classified under the Japanese Committee's Classification.

2. A pharmaceutical preparation for preventing and treating bacterial infections, which comprises the human monoclonal antibody of Claim 1.

3. The preparation of Claim 2, which is further used together with antibiotics.

4. The preparation of Claim 3, in which the antibiotics are carbapenem or aminoglycoside antibiotics.

5. A hybridoma cell line or its descendant cell line which produces the human monoclonal antibody of Claim 1.

6. A process for preparing the human monoclonal antibody of Claim 1, which comprises culturing the hybridoma cell line or its descendant cell line of Claim 5, and recovering the produced antibody from the culture.

7. The preparation of any of Claims 2 to 4, which additionally comprises:

1) a monoclonal antibody capable of recognizing the outer core of LPS of P. aeruginosa, which antibody shows synergistic effects on experimental P. aeruginosa infections in association with carbapenem or aminoglycoside antibiotics; and/or

2) a monoclonal antibody capable of recognizing the flagellum protein of P. aeruginosa, which antibody shows synergistic effects on experimental P. aeruginosa infections in association with carbapenem or aminoglycoside antibiotics.

8. A pharmaceutical preparation for preventing and treating an endotoxin shock caused by P. aeruginosa, which comprises the human monoclonal antibody of Claim 1.

9. A method for the preparation of a pharmaceutical preparation according to claim 2 or 8, which comprises formulating the monoclonal antibody of claim 1 with a pharmaceutically acceptable carrier, or according to claim 7, which comprises formulating the monoclonal antibody of claim 1 and

1) a monoclonal antibody capable of recognizing the outer core of LPS of P. aeruginosa, which antibody shows synergistic effects on experimental P. aeruginosa infections in association with carbapenem or aminoglycoside antibiotics; and/or

2) a monoclonal antibody capable of recognizing the flagellum protein of P. aeruginosa, which antibody shows synergistic effects on experimental P. aeruginosa infections in association with carbapenem or aminoglycoside antibiotics

with a pharmaceutically acceptable carrier.

10. A method for the preparation of a pharmaceutical preparation according to claim 3 or 4, which comprises formulating the human monoclonal antibody of claim 1 with an antibiotic, preferentially with carbapenem or an aminoglycoside antibiotic, or according to claim 7, wherein

1) a monoclonal antibody capable of recognizing the outer core of LPS of P. aeruginosa, which antibody shows synergistic effects on experimental P. aeruginosa infections in association with carbapenem or aminoglycoside antibiotics; and/or

2) a monoclonal antibody capable of recognizing the flagellum protein of P. aeruginosa, which antibody shows synergistic effects on experimental P. aeruginosa infections in association with carbapenem or aminoglycoside antibiotics

are additionally included into the preparation.

EP 0 441 395 B1

**Patentansprüche**

1. Menschlicher monoclonaler Antikörper, erhältlich von der Hybridomzellinie HI-223, die bei dem Fermentation Research Institute, Agency of Industrial Science and Technology unter den Bedingungen des Budapester Vertrages unter der Hinterlegungsnummer FERM BP-3213 hinterlegt wurde, oder von einer davon abstammenden Zellinie, wobei der menschliche monoclonale Antikörper die folgenden Eigenschaften aufweist:

   1) die antigene Determinante für den monoclonalen Antikörper ist ein O-Antigen von P. aeruginosa;

   2) der monoclonale Antikörper ist prophylaktisch und therapeutisch wirksam bei Infektionen, die durch P. aeruginosa verursacht werden;

   3) der monoclonale Antikörper zeigt synergistische Wirkungen bei der Behandlung von experimentellen P. aeruginosa-Infektionen, wenn er zusammen mit Carbapenem oder Aminoglycosid-Antibiotika verwendet wird;

   4) der monoclonale Antikörper gehört zur IgM (Immunglobulin M)-Klasse; und

   5) der menschliche monoclonale Antikörper erkennt Lipopolysaccharide von P. aeruginosa-Stämmen vom Serotyp E, klassifiziert gemäß der Klassifizierung des Japanischen Komitees.

2. Arzneimittel zur Vorbeugung gegen und Behandlung von bakteriellen Infektionen, umfassend den menschlichen monoclonalen Antikörper nach Anspruch 1.

3. Mittel nach Anspruch 2, das zusammen mit Antibiotika verwendet wird.

4. Mittel nach Anspruch 3, wobei die Antibiotika Carbapenem oder Aminoglycosid-Antibiotika sind.

5. Hybridomzellinie oder eine davon abstammende Zellinie, die den menschlichen monoclonalen Antikörper nach Anspruch 1 produziert.

6. Verfahren zur Herstellung des menschlichen monoclonalen Antikörpers nach Anspruch 1, umfassend die Züchtung der Hybridomzellinie oder der davon abstammenden Zellinie nach Anspruch 5 und die Gewinnung des produzierten Antikörpers aus der Kultur.

7. Mittel nach einem der Ansprüche 2 bis 4, zusätzlich umfassend:

   1) einen monoclonalen Antikörper, der die äußere Hülle aus LPS von P. aeruginosa erkennen kann, wobei der Antikörper synergistische Effekte bei experimentellen P. aeruginosa-Infektionen in Verbindung mit Carbapenem oder Aminoglycosid-Antibiotika zeigt; und/oder

   2) einen monoclonalen Antikörper, der das Flagellum-Protein von P. aeruginosa erkennen kann, wobei der Antikörper synergistische Effekte bei experimentellen P. aeruginosa-Infektionen in Verbindung mit Carbapenem oder Aminoglycosid-Antibiotika zeigt.

8. Arzneimittel zur Vorbeugung und Behandlung eines durch P. aeruginosa verursachten Endotoxin-Schocks, umfassend den menschlichen monoclonalen Antikörper nach Anspruch 1.

9. Verfahren zur Herstellung eines Arzneimittels nach Anspruch 2 oder 8, umfassend die Formulierung des monoclonalen Antikörpers nach Anspruch 1 mit einem pharmazeutisch verträglichen Träger, oder nach Anspruch 7, umfassend die Formulierung des monoclonalen Antikörpers nach Anspruch 1 und

   1) eines monoclonalen Antikörpers, der die äußere Hülle aus LPS von P. aeruginosa erkennen kann, wobei der Antikörper synergistische Effekte bei experimentellen P. aeruginosa-Infektionen in Verbindung mit Carbapenem oder Aminoglycosid-Antibiotika zeigt; und/oder

   2) eines monoclonalen Antikörpers, der das Flagellum-Protein von P. aeruginosa erkennen kann, wobei der Antikörper synergistische Effekte bei experimentellen P. aeruginosa-Infektionen in Verbindung mit Carbapenem oder Aminoglycosid-Antibiotika zeigt,

   mit einem pharmazeutisch verträglichen Träger.

10. Verfahren zur Herstellung eines Arzneimittels nach Anspruch 3 oder 4, umfassend die Formulierung des menschlichen monoclonalen Antikörpers nach Anspruch 1 mit einem Antibiotikum, vorzugsweise mit Carbapenem oder einem Aminoglycosid-Antibiotikum, oder nach Anspruch 7, wobei

   1) ein monoclonaler Antikörper, der die äußere Hülle aus LPS von P. aeruginosa erkennen kann, wobei der Antikörper synergistische Effekte bei experimentellen P. aeruginosa-Infektionen in Verbindung mit Carbapenem oder Aminoglycosid-Antibiotika zeigt; und/oder

2) ein monoclonaler Antikörper, der das Flagellum-Protein von P. aeruginosa erkennen kann, wobei der Antikörper synergistische Effekte bei experimentellen P. aeruginosa-Infektionen in Verbindung mit Carbapenem oder Aminoglycosid-Antibiotika zeigt,

zusätzlich in das Mittel eingebracht werden.

**Revendications**

1. Anticorps monoclonal humain qui peut être obtenu à partir de la lignée cellulaire d'hybridome HI-223, qui a été déposée auprès du Fermentation Research Institute, Agency of Industrial Science and Technology en vertu du traité de Budapest sous le n° de dépôt FERM BP-3213, ou de sa lignée cellulaire descendante, ledit anticorps monoclonal humain ayant les caractéristiques suivantes :

   1) le déterminant antigénique pour l'anticorps monoclonal est un antigène O de P. aeruginosa;

   2) l'anticorps monoclonal est efficace du point de vue prophylactique et thérapeutique sur les infections provoquées par P. aeruginosa;

   3) l'anticorps monoclonal présente des effets de synergie dans le traitement des infections à P. aeruginosa expérimentales lorsqu'il est utilisé en association avec des antibiotiques de type carbapenem ou aminoglycoside;

   4) l'anticorps monoclonal appartient à la classe des IgM (immunoglobulines M) ; et

   5) l'anticorps monoclonal humain reconnait les lipopolysaccharides des souches de P. aeruginosa de sérotype E classé selon la Japanese Committee's Classification.

2. Préparation pharmaceutique pour prévenir et traiter les infections bactériennes, qui comprend l'anticorps monoclonal humain de la revendication 1.

3. Préparation selon la revendication 2, qui est utilisée en outre avec des antibiotiques.

4. Préparation selon la revendication 3, dans laquelle les antibiotiques sont des antibiotiques de type carbapenem ou aminoglycoside.

5. Lignée cellulaire d'hybridome ou sa lignée cellulaire descendante qui produit l'anticorps monoclonal humain de la revendication 1.

6. Procédé pour préparer l'anticorps monoclonal humain de la revendication 1, qui comprend la culture de la lignée cellulaire d'hybridome ou de sa lignée cellulaire descendante de la revendication 5, et la récupération de l'anticorps produit à partir de la culture.

7. Préparation selon l'une quelconque des revendications 2 à 4, qui comprend en outre :

   1) un anticorps monoclonal capable de reconnaître le coeur externe de LPS de P. aeruginosa lequel anticorps présente des effets de synergie sur les infections à P. aeruginosa expérimentales en association avec des antibiotiques de type carbapenem ou aminoglycoside ; et/ou

   2) un anticorps monoclonal capable de reconnaître la protéine de flagelle de P. aeruginosa, lequel anticorps présente des effets de synergie sur les infections à P. aeruginosa expérimentales en association avec des antibiotiques de type carbapenem ou aminoglycoside.

8. Préparation pharmaceutique pour prévenir et traiter un choc endotoxinique provoqué par P. aeruginosa, qui comprend l'anticorps monoclonal humain de la revendication 1.

9. Procédé pour la préparation d'une préparation pharmaceutique selon la revendication 2 ou 8, qui comprend la formulation de l'anticorps monoclonal de la revendication 1 avec un véhicule pharmaceutiquement acceptable, ou selon la revendication 7, qui comprend la formulation de l'anticorps monoclonal de la revendication 1 et

   1) d'un anticorps monoclonal capable de reconnaître le coeur externe de LPS de P. aeruginosa, lequel anticorps présente des effets de synergie sur les infections à P. aeruginosa expérimentales en association avec des antibiotiques de type carbapenem ou aminoglycoside ; et/ou

   2) d'un anticorps monoclonal capable de reconnaître la protéine de flagelle de P. aeruginosa lequel anticorps présente des effets de synergie sur les infections à P. aeruginosa expérimentales en association avec des antibiotiques de type carbapenem ou aminoglycoside

avec un véhicule pharmaceutiquement acceptable.

10. Procédé pour la préparation d'une préparation pharmaceutique selon la revendication 3 ou 4, qui comprend la formulation de l'anticorps monoclonal humain de la revendication 1 avec un antibiotique, de préférence avec un antibiotique de type carbapenem ou aminoglycoside, ou selon la revendication 7, dans lequel

    1) un anticorps monoclonal capable de reconnaître le coeur externe de LPS de P. aeruginosa, lequel anticorps présente des effets de synergie sur les infections à P. aeruginosa expérimentales en association avec des antibiotiques de type carbapenem ou aminoglycoside ; et/ou

    2) un anticorps monoclonal capable de reconnaître la protéine de flagelle de P. aeruginosa, lequel anticorps présente des effets de synergie sur les infections à P. aeruginosa expérimentales en association avec des antibiotiques de type carbapenem ou aminoglycoside

sont inclus en outre dans la préparation.